# Europäisches Patentamt

## European Patent Office

## Office européen des brevets

(11) Veröffentlichungsnummer: **0 092 185 B1**

(12) **EUROPÄISCHE PATENTSCHRIFT**

(45) Veröffentlichungstag der Patentschrift: **20.04.88**

(21) Anmeldenummer: **83103632.2**

(22) Anmeldetag: **14.04.83**

(51) Int. Cl.⁴: **C 07 D 401/06, A 61 K 31/47**

(54) Mefloquin-hydrochlorid und Verfahren zu seiner Reindarstellung.

(30) Priorität: **14.04.82 CH 2256/82**

(43) Veröffentlichungstag der Anmeldung:
**26.10.83 Patentblatt 83/43**

(45) Bekanntmachung des Hinweises auf die Patenterteilung:
**20.04.88 Patentblatt 88/16**

(84) Benannte Vertragsstaaten:
**AT BE CH DE FR GB IT LI NL**

(56) Entgegenhaltungen:
**EP - A - 0 026 894**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Bömches, Helmut, Käppelirainweg 26, CH-4147 Aesch (CH)**
Erfinder: **Hardegger, Bruno, Dr., Bäumliweg 36, CH-4125 Riehen (CH)**

(74) Vertreter: **Lederer, Franz, Dr. et al, Van der Werth, Lederer & Riederer Patentanwälte Lucile-Grahn-Strasse 22, D-8000 München 80 (DE)**

## Beschreibung

Mefloquin, erythro-α-2-Piperidyl-2,8-bis-(trifluormethyl)-4-chinolinmethanol, ist ein wertvoller Wirkstoff zur Behandlung auch der chloroquinresistenten Form der Malaria (vgl. z.B. Antimicrobial Agents Chemother. 9, 384 [1976]), für dessen Herstellung mehrere Verfahren bekannt sind (vgl. J. Med. Chem. 14, 926 [1971]; DOS 2 806 909; DOS 2 940 443). Bei denjenigen Verfahren zur Herstellung von Mefloquin, deren letzte Stufe in der katalytischen Hydrierung des 2-Pyridyl-2,8-bis-(trifluormethyl)-4-chinolylketons besteht, fällt neben der biologisch aktiven erythro-Form auch stets eine geringere Menge (ca. 5–15%) der inaktiven und daher unerwünschten threo-Form an. Die Trennung dieses Gemisches und die Reindarstellung der erythro-Form war bisher nur durch ein relativ aufwendiges Verfahren, das die mehrmalige Umkristallisation aus einem Aceton/Alkohol-Gemisch, Waschen mit Aceton und Kristallisation aus Acetonitril umfasste, möglich.

Es wurde nun gefunden, dass die Abtrennung der threo- von der erythro-Form und die Reindarstellung der letzteren sich in überraschend einfacher Weise dadurch erreichen lässt, dass man das bei der Hydrierung anfallende Gemisch von erythro- und threo-α-2-Piperidyl-2,8-bis-(trifluormethyl)-4-chinolinmethanol-hydrochlorid mit wässrigem Methanol oder Ethanol behandelt, wobei die threo-Form in Lösung geht und die erythro-Form zurückbleibt. Reste der threo-Verbindung sowie anderer Nebenprodukte lassen sich durch weitere Reinigung, beispielsweise durch Behandlung mit Aceton (d.h. via Aceton-Komplex), entfernen.

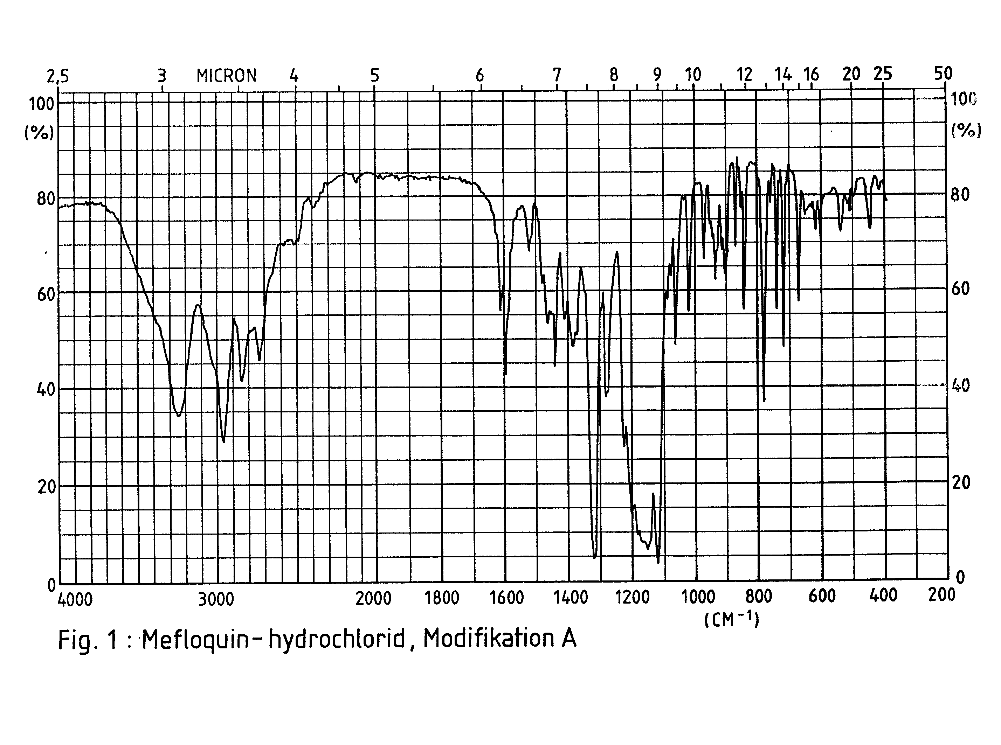

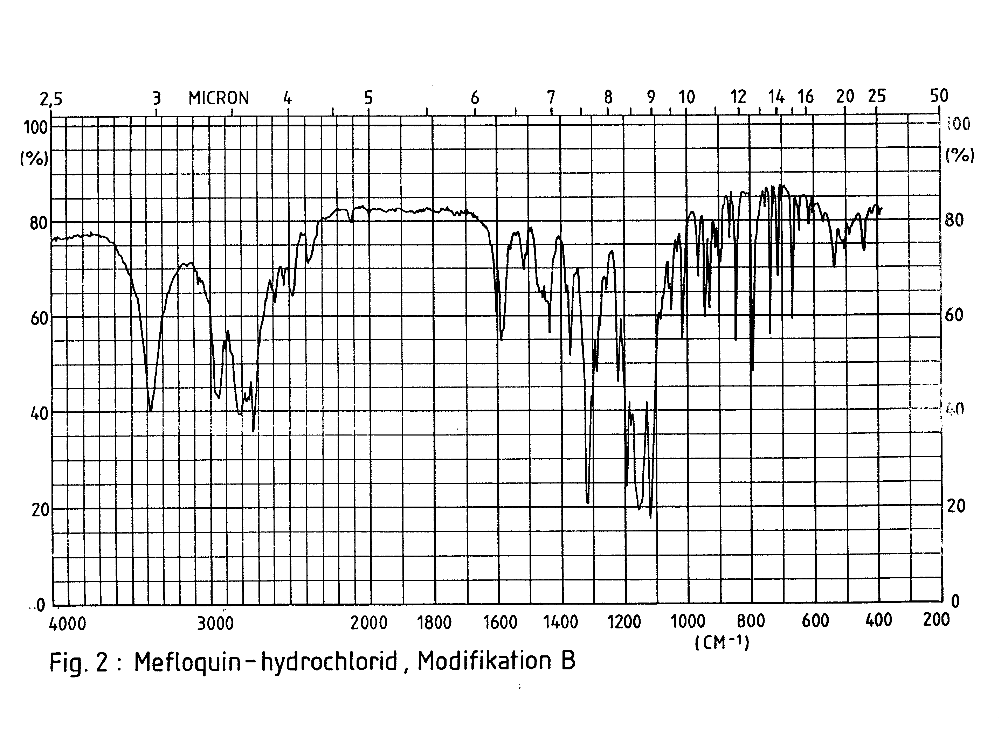

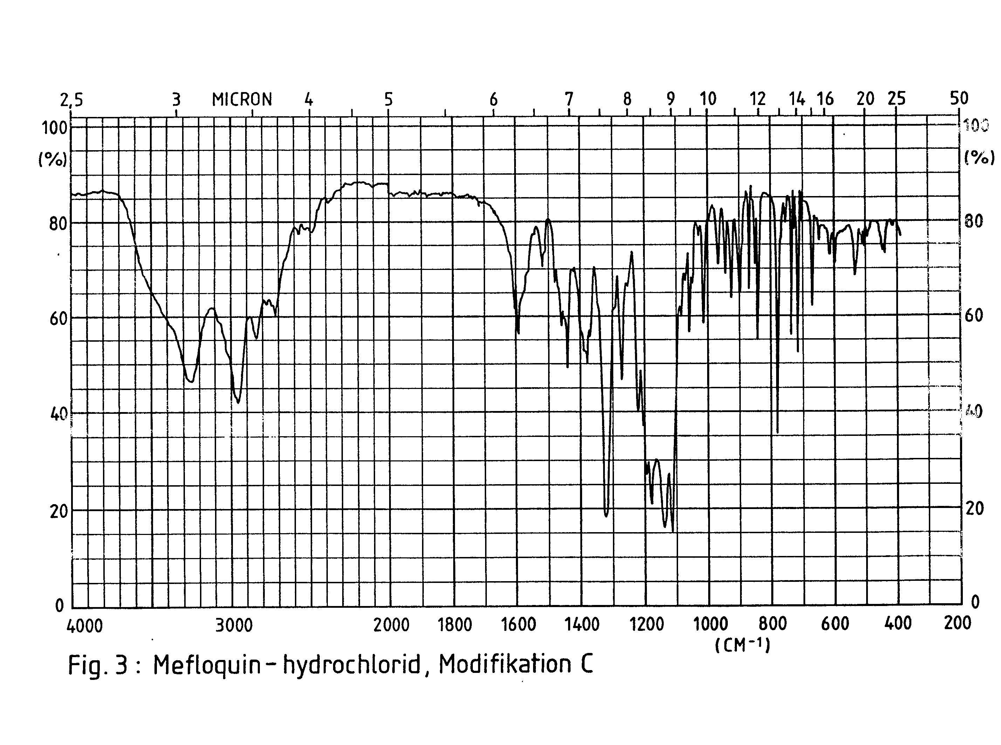

Es wurde ferner gefunden, dass reines Mefloquin-hydrochlorid in verschiedenen Modifikationen vorkommt, die sich kristallographisch oder aufgrund ihrer IR-Spektren voneinander unterscheiden lassen. So erhält man bei der Reinigung mittels Acetonitril eine Modifikation A, Mefloquin · HCl, die durch das IR-Spektrum A (Figur 1) charakterisiert ist, während bei kurzzeitiger Behandlung mit Aceton die Modifikation B, Mefloquin · HCl · Aceton, und mit Wasser/Alkohol-Gemischen die Modifikation C, Mefloquin · HCl · 1/2H₂O, erhalten wird (vgl. IR-Spektren B und C, Figuren 2 und 3). Bei längerer Behandlung von Mefloquin-hydrochlorid mit Alkohol/Wasser-Gemischen bildet sich die Modifikation D, Mefloquin · HCl · 1/2H₂O, die die thermodynamisch stabilste ist (vgl. IR-Spektrum D, Figur 4). Ein Vergleich der IR-Spektren zeigt, dass die Unterschiede in den Wellenzahl-Bereichen 900–960 und 1100–1200 cm⁻¹ liegen.

Die vorliegende Erfindung betrifft daher ein Verfahren zur Gewinnung von erythro-α-2-Piperidyl-2,8-bis-(trifluormethyl)-4-chinolinmethanol-hydrochlorid aus Gemischen von erythro- und threo-α-2-Piperidyl-2,8-bis-(trifluormethyl)-4-chinolinmethanol-hydrochlorid, insbesondere aus solchen, wie sie bei der Hydrierung von 2-Pyridyl-2,8-bis-(trifluormethyl)-4-chinolyl-keton erhalten werden, durch Behandlung dieser Gemische mit wässrigem Methanol oder Ethanol.

Die Erfindung betrifft ferner erythro-α-2-Piperidyl-2,8-bis-(trifluormethyl)-4-chinolinmethanol-hydrochlorid in Form seiner thermodynamisch stabilsten Modifikation, aus Alkohol/Wasser-Gemischen kristallisiert, charakterisiert durch sein IR-Spektrum, sowie dessen Herstellung durch langzeitige Behandlung von mindestens teilweise in anderer Modifikation vorliegendem Mefloquin-hydrochlorid mit einem Alkohol/Wasser-Gemisch. Bei der Behandlung von erythro-/threo-α-2-Piperidyl-2,8-bis-(trifluormethyl)-4-chinolinmethanol-Gemischen mit wässrigem Methanol oder Ethanol geht die threo-Form in Lösung. Die Behandlung kann durch 30minütiges bis mehrstündiges Rühren bei Zimmertemperatur oder unter Erwärmen bis auf etwa 80°C und anschliessende Abkühlung auf ca. 5°C (zur Erhöhung der Ausbeute) erfolgen. Der Wasseranteil am Lösungsmittelgemisch kann in weiten Grenzen variiert werden und liegt zweckmässigerweise bei 60–90 Vol.-%, vorzugsweise zwischen 70 und 90 Vol.-%. Bei der Aufarbeitung methanolischer Reaktionslösungen, wie sie beispielsweise bei der katalytischen Hydrierung von 2-Pyridyl-2,8-bis-(trifluormethyl)-4-chinolylketon in salzsaurem Methanol erhalten werden, wird zweckmässigerweise zunächst ein Teil des Methanols abgezogen und dann eine entsprechende Menge Wasser zugesetzt. Das so erhaltene Mefloquin-hydrochlorid (Rohware) wird zweckmässigerweise noch mit kaltem Wasser gewaschen und ist dann praktisch frei von der unerwünschten threo-Form.

Durch Aufarbeitung der Mutterlaugen nach der vorstehend beschriebenen Methode in Kombination mit der Methode der Aceton-Komplex-Bildung kann prinzipiell eine vollständige Trennung von erythro- und threo-Form erreicht werden.

Die so erhaltene Rohware ist chemisch zwar praktisch einheitlich, liegt im allgemeinen aber weder in einer einheitlichen Kristallform noch in der thermodynamisch stabilsten Form vor. Um dies zu erreichen, wird die Rohware längere Zeit, d.h. mindestens etwa 6 Stunden bis zu etwa 12 Stunden mit einem Alkohol, z.B. Methanol, Ethanol, Isopropanol, vorzugsweise aber Methanol, in Gegenwart von Wasser (ca. 50–80 Vol.-%) verrührt. Die Behandlung kann bei Zimmertemperatur oder unter Kühlung auf Temperaturen etwas über 0°C – zur Erhöhung der Ausbeute – erfolgen.

Beispiel 1

50 kg 2-Pyridyl-2,8-bis-(trifluormethyl)-4-chinolylketon wurden in 550 l Methanol unter Erwärmen auf 56–58°C gelöst. Die Lösung wurde auf 25°C abgekühlt und mit einer vorhydrierten Suspension von 2,5 kg Platindioxid und 2,5 kg Hydrierkohle in 210 l Methanol und 14,1 l 33%iger eisenfreier Salzsäure (d = 1,170) vereinigt. Es wurde 6–8 Stunden unter Normaldruck bei einer maximalen Innentemperatur von 30°C bis zur Beendigung der Wasserstoffaufnahme hydriert.

Das Reaktionsgemisch wurde filtriert, die methanolische Lösung unter vermindertem Druck auf ein Volumen von 100–150 l eingeengt, mit 250–350 l Wasser versetzt und anschliessend wieder unter vermindertem Druck auf ein Volumen von 300–350 l gebracht. Man rührte 1 Stunde unter langsamem Erwärmen auf 80 °C, dann weitere 30 Minuten bei dieser Temperatur, kühlte auf 5 °C ab und rührte eine weitere Stunde. Die Suspension wurde zentrifugiert, das noch feuchte Schwinggut in 250 l Wasser von 5 °C eingebracht und 15 Minuten gerührt. Dann wurde wieder zentrifugiert, das Schwinggut noch mehrmals mit je 20 l Wasser von 5 °C abgebraust, gut ausgeschwungen und 12 Stunden bei 70 °C unter vermindertem Druck getrocknet. Man erhielt 46–48 kg (82,1–85,6% d. Th.) rohes Mefloquin-hydrochlorid, das praktisch frei von der unerwünschten threo-Form war und das wie folgt weitergereinigt wurde:

Eine Supsension von 150 kg rohem Mefloquin-hydrochlorid in 75 l absolutem Ethanol und 425 l Aceton wurde unter Rühren innerhalb einer Stunde auf Rückflusstemperatur gebracht und 30 Minuten bei dieser Temperatur gehalten. Es wurde auf 20 °C abgekühlt, mit 50 l eines Ethanol/Aceton-Gemisches (42,5:7,5) versetzt, unter Rühren auf ca. 0 °C abgekühlt und 4 Stunden bei dieser Temperatur nachgerührt. Die Suspension wurde zentrifugiert, das gut ausgeschwungene Gut mehrmals mit je 15 l Aceton von 5 °C abgebraust und im Vakuumtrockner bei 70 °C getrocknet. Man erhielt 136–142 kg gereinigtes Mefloquin-hydrochlorid.

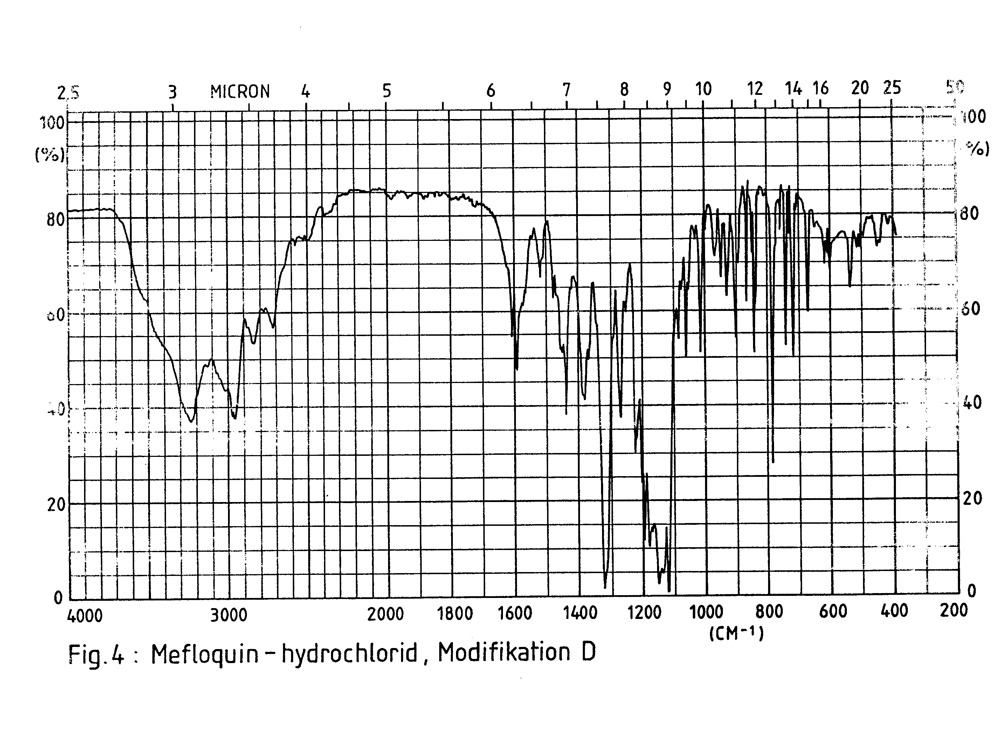

100 kg von so gereinigtem Mefloquin-hydrochlorid in 300 l Methanol wurden zum Rückfluss erhitzt. Die heisse Lösung wurde in 1180 l Wasser eingebracht und mindestens 4 Stunden gerührt. Unter Kühlung auf 2 °C wurde dann noch mindestens 6 Stunden nachgerührt. Nach Zentrifugieren und zweimaligem Abbrausen des Schwinggutes mit je 15 l Wasser von 5 °C wurde im Vakuumtrockner bei 70 °C getrocknet. Man erhielt 80–85 kg reines Mefloquin-hydrochlorid in seiner aus wässrigen Medien anfallenden thermodynamisch stabilsten Modifikation, Smp. 255–259 °C (vgl. IR-Spektrum D, Figur 4). Durch Extraktion der Mutterlauge mit Methylenchlorid und Aufarbeitung der organischen Phase im Labor kann die Ausbeute an reinem Produkt noch erhöht werden.

Beispiel 2

120 kg 2-Pyridyl-2,8-bis-(trifluormethyl)-4-chinolylketon wurden in 196 l Methanol und 17,4 kg 30%iger Salzsäure suspendiert und mit einer vorhydrierten Suspension von 9 kg Platindioxid und 9 kg Hydrierkohle in 300 l Methanol vereinigt. Es wurde 10 Stunden unter 0,1–0,5 bar Überdruck bei einer maximalen Innentemperatur von 20 °C bis zur Beendigung der Wasserstoffaufnahme hydriert.

Das Reaktionsgemisch wurde filtriert, Kessel und Filter mit 80 l Methanol nachgespült, die methanolische Lösung unter vermindertem Druck auf ein Volumen von 100–150 l eingeengt und mit 800–880 l Wasser versetzt. Man rührte 1 Stunde unter langsamem Erwärmen auf 80 °C, dann weitere 30 Minuten bei dieser Temperatur, kühlte auf 5 °C ab und rührte eine weitere Stunde. Die Suspension wurde zentrifugiert, das Schwinggut noch mehrmals mit je 30 l Wasser von 5 °C abgebraust, gut ausgeschwungen und 12 Stunden bei 80 °C unter vermindertem Druck getrocknet. Man erhielt 110–115 kg rohes Mefloquin-hydrochlorid, das praktisch frei von der unerwünschten threo-Form war und – wie in Beispiel 1 beschrieben – weitergereinigt wurde.

Beispiel 3
Tablette enthaltend

| | |
|---|---|
| Mefloquin-hydrochlorid, Mod. D | 274,12 mg |
| Cellulose | 100,0 mg |
| D-Mannit | 87,88 mg |
| Polyvinylpyrrolidon | 15,0 mg |
| Natriumcarboxymethylstärke | 10,0 mg |
| Talk | 10,0 mg |
| Magnesiumstearat | 3,0 mg |
| | 500,0 mg |

Herstellung:

Eine gesiebte Mischung von Mefloquin-hydrochlorid (Mod. D), D-Mannit pulv. und mikrokristalliner Cellulose (Teil) wird mit einer wässrigen Lösung von Polyvinylpyrrolidon befeuchtet und geknetet. Sodann wird granuliert, getrocknet und gesiebt. Dem Granulat wird eine gesiebte Mischung von mikrokristalliner Cellulose (Teil), Natriumcarboxymethylstärke, Talk und Magnesiumstearat zugesetzt. Sämtliche Pulverbestandteile werden miteinander gemischt, und das homogene Pressgut wird zu Kernen mit einem Gewicht von 500 mg und einer Härte von mindestens 10 SCE gepresst.

**Patentansprüche**

1. Verfahren zur Gewinnung von Mefloquin-hydrochlorid aus Gemischen von erythro- und threo-α-2-Piperidyl-2,8-bis-(trifluormethyl)-4-chino-linmethanol-hydrochlorid, dadurch gekennzeichnet, dass man diese Gemische mit wässrigem Methanol oder Ethanol behandelt.

2. Verfahren gemäss Anspruch 1, dadurch gekennzeichnet, dass das Gemisch ein solches ist, wie es durch Hydrierung von 2-Pyridyl-2,8-bis-(trifluormethyl)-4-chinolyl-keton erhalten wird.

3. Verfahren gemäss Anspruch 1 oder 2, dadurch gekennzeichnet, dass das wässrige Methanol oder Aethanol etwa 70–90 Vol.-% Wasser enthält.

4. Verfahren zur Gewinnung von reinem Mefloquin-hydrochlorid in Form seiner thermodynamisch stabilsten Modifikation, dadurch gekennzeichnet, dass man Mefloquin-hydrochlorid, das mindestens teilweise in anderer Modifikation vorliegt, während mindestens 6 Stunden mit einem Alkohol/Wasser-Gemisch behandelt.

5. Verfahren gemäss Anspruch 4, dadurch gekennzeichnet, dass die Behandlung bei einer Temperatur zwischen 0 °C und Zimmertemperatur erfolgt.

6. Reines Mefloquin-hydrochlorid in seiner thermodynamisch stabilsten Modifikation, erhältlich gemäss Anspruch 4.

7. Pharmazeutische Präparate auf der Basis von reinem Mefloquin-hydrochlorid in seiner thermodynamisch stabilsten Modifikation, erhältlich gemäss Anspruch 4.

**Claims**

1. A method for obtaining mefloquin hydrochloride from mixtures of erythro- and threo-α-2-piperydil-2,8-bis-(trifluoromethyl)-4-quinoli-nemethanol hydrochloride, characterized by treating these mixtures with aqueous methanol or ethanol.

2. A method in accordance with claim 1, characterized in that the mixture is one which has been obtained by hydrogenating 2-pyridyl 2-8-bis-(trifluoromethyl)-4-quinolyl ketone.

3. A method in accordance with claim 1 or 2, characterized in that the aqueous methanol or ethanol contains about 70–90 vol.% water.

4. A method for preparing pure mefloquin hydrochloride in the form of its thermodynamically most stable modification, characterized by treating mefloquin hydrochloride, which is present at least partially in another modification, with an alcohol/water mixture for at least 6 hours.

5. A method in accordance with claim 4, characterized in that the treatment is effected at a temperature between 0 °C and room temperature.

6. Pure mefloquin hydrochloride in its thermodynamically most stable modification, obtainable in accordance with claim 4.

7. A pharmaceutical preparation based on pure mefloquin hydrochloride in its thermodynamically most stable modification, obtainable in accordance with claim 4.

**Revendications**

1. Procédé d'obtention de chlorhydrate de méfloquine à partir de mélanges de chlorhydrate de α-2-pipéridyl-2,8-bis-(trifluorométhyl)-4-quino-léineméthanol érythro et thréo caractérisé en ce qu'on traite ces mélanges avec du méthanol ou de l'éthanol aqueux.

2. Procédé selon la revendication 1, caractérisé en ce que le mélange est un mélange tel qu'obtenu par hydrogénation de 2-pyridyl-2,8-bis-(trifluorométhyl)-4-quinoléine-cétone.

3. Procédé selon la revendication 1 ou 2, caractérisé en ce que le méthanol ou l'éthanol aqueux contient environ 70–90% en volume d'eau.

4. Procédé d'obtention de chlorhydrate de méfloquine pur sous la forme de sa forme la plus stable thermodynamiquement, caractérisé en ce qu'on traite le chlorhydrate de méfloquine, qui se présente au moins en partie sous une autre forme, pendant au moins 6 heures avec un mélange alcool/eau.

5. Procédé selon la revendication 4, caractérisé en ce qu'on conduit le traitement à une température comprise entre 0 °C et la température ambiante.

6. Chlorhydrate de méfloquine pur sous sa forme la plus stable thermodynamiquement que l'on peut obtenir selon la revendication 4.

7. Préparations pharmaceutiques à base de chlorhydrate de méfloquine pur sous sa forme la plus stable thermodynamiquement, que l'on peut obtenir selon la revendication 4.

Fig. 1 : Mefloquin-hydrochlorid , Modifikation A

Fig. 2 : Mefloquin-hydrochlorid, Modifikation B

Fig. 3 : Mefloquin — hydrochlorid, Modifikation C

Fig.4 : Mefloquin - hydrochlorid , Modifikation D